# EUROPÄISCHE PATENTSCHRIFT

(11) **EP 0 960 093 B1**
(45) Veröffentlichungstag und Bekanntmachung des Hinweises auf die Patenterteilung: **22.08.2001**
(21) Anmeldenummer: 98905350.9
(22) Anmeldetag: 26.01.1998
(51) Int. Cl.: C07C 209/52, C07C 209/70, C07C 211/12, C07C 209/84

(54) **VERFAHREN ZUR HYDRIERUNG VON VERBINDUNGEN AUS DER GRUPPE DER IMINE ODER ENAMINE**
METHOD FOR HYDROGENATING COMPOUNDS FROM THE GROUP OF IMINES OR ENAMINES
PROCEDE D'HYDROGENATION DE COMPOSES DU GROUPE DES IMINES OU DE ENAMINES

(30) Priorität: 07.02.1997 DE 19704615
(43) Veröffentlichungstag der Anmeldung: 01.12.1999
(73) Patentinhaber: BASF AKTIENGESELLSCHAFT, 67056 Ludwigshafen (DE)
(72) Erfinder: FUCHS, Eberhard, D-67227 Frankenthal (DE); OHLBACH, Frank, D-56370 Dörsdorf (DE)
(86) Internationale Anmeldenummer: EP9800414
(87) Internationale Veröffentlichungsnummer: WO9834899

(56) Entgegenhaltungen:
- EP-A- 0 497 333
- EP-A- 0 502 439
- DE-A- 4 446 893
- DE-C- 19 540 191
- DE-C- 19 630 788

## Beschreibung

Die vorliegende Erfindung betrifft ein Verfahren zur Hydrierung einer Verbindung aus der Gruppe der Imine oder Enamine I zu einem Amin II in Gegenwart eines Nitrils III, wobei das Nitril III im wesentlichen nicht hydriert wird, dadurch gekennzeichnet, daß man eine Mischung enthaltend Tetrahydroazepin als Verbindung I und 6-Aminocapronitril als Nitril III in Gegenwart eines Katalysators IV mit einem molekularen Wasserstoff enthaltenden Gas umsetzt.

Ferner betrifft die vorliegende Erfindung die Verwendung von Katalysatoren zur Umsetzung von Iminen oder Enaminen zu Aminen in Gegenwart von Nitrilen, wobei die Nitrile im wesentlichen nicht hydriert werden, mit einem molekularen Wasserstoff enthaltenden Gas.

Verfahren zur Hydrierung von Iminen oder Enaminen in Gegenwart von Nitrilen, wobei die Nitrile im wesentlichen nicht hydriert werden, sind bekannt.

Aus EP-A-0 502 439 ist bekannt, ein Imin wie Tetrahydroazepin in Gegenwart eines Nitrils wie 6-Aminocapronsäurenitril mit einem erheblichen Überschuß an Hydriden wie Natriumborhydrid oder Lithium-tri-tert.-butoxy-aluminiumhydrid zu N-(5-cyanopentyl)-1,6-hexamethylendiamin umzusetzen. Nachteilig ist bei diesem Verfahren, daß das Hydrid in vier- bis fünffachem Überschuß eingesetzt wird und nach der Umsetzung das aus dem Hydrid entstandene Salz abgetrennt werden muß. Zudem muß das auf die Hydrierung folgende Verfahren zur Aufarbeitung des Produktgemisches auf das verbleibende Hydrid und die gesamten Produkte jeweils abgestimmt werden, um eine Hydrierung des Nitrils bei der Aufarbeitung zu vermeiden.

DE-A-19 630 788 offenbart ein Verfahren (Beispiel 3) worin Adipodinitril (ADN) mit mit NH₃ und H₂ über einem Katalysator zu einem Gemisch von Adipodinitril, Aminocapronitril und Hexamethylendiamin (HMDA) hydriert wird. Aus ADN und NH wird in situ ein Imin gebildet. Diese Umsetzung ist nicht vollständig, was daraus abgeleitet werden kann, daß das Produkt noch ADN enthält. In situ wird also ein Gemisch aus Imin und Nitril mit H₂ in Anwesenheit von einem Katalysator zum Amin hydriert.

DE-A-19 540 191 offenbart ein Verfahren zur Herstellung von Isophorondiamin. Hierzu wird Isophorondinitril mit NH₃ und Methanol "unvollständig" zum Isophoroniminonitril umgesetzt. Diese Mischung (Isophorondinitril zusammen mit Isophoroniminonitril) wird dann unter Verwendung eines Hydrierkatalysators mit Wasserstoff zu Isophorondiamin umgesetzt.

DE-A-19 636 766 offenbart ein Verfahren zur gleichzeitigen Herstellung von 6-Aminocapronitril (6-ACN) und Hexamehtylendiamin (HMDA). Aus der Figur ist deutlich, daß Adipodinitril (ADN) mit NH₃ umgesetzt wird. Durch diese Umsetzung kann in situ ein Gemisch aus ADN zusammen mit ein Imin erhalten werden, das dann mit Wasserstoff weiterhydriert wird.

EP-A-0 497 333 offenbart ein Verfahren worin ein Gemisch, das ein Nitril (ACN) und ein Imin (THA) enthält, mit eine Base gereinigt wird (siehe Beispiel 1).

Der vorliegenden Erfindung liegt die Aufgabe zugrunde, ein Verfahren zur Verfügung zu stellen, das die genannten Nachteile vermeidet und die Hydrierung von Iminen zu Aminen in Gegenwart von Nitrilen, wobei die Nitrile im wesentlichen nicht hydriert werden, auf technisch einfache und wirtschaftliche Weise ermöglicht. Demgemäß wurde das eingangs genannte Verfahren und die Verwendung von Katalysatoren für dieses Verfahren gefunden.

Als Verbindung I kommen Tetrahydroazepin, wie 3,4,5,6-Tetrahydroazepin oder 4,5,6,7-Tetrahydroazepin sowie deren Gemische in Betracht.

Solche Tetrahydroazepine sowie Verfahren zu deren Herstellung sind allgemein bekannt.

Diese Tetrahydroazepine können als einzelne Verbindungen vorliegen oder Addukte, insbesondere mit Aminen, bilden, wobei diese Addukte im Sinne der vorliegenden Erfindung ebenfalls als Tetrahydroazepin bezeichnet werden.

Tetrahydroazepin kann bei der partiellen katalytischen Hydrierung mit einem molekularen Wasserstoff enthaltenden Gas von Adipodinitril zu 6-Aminocapronsäurenitril oder Mischungen, die 6-Aminocapronsäurenitril und Hexamethylendiamin enthalten, in der Regel in Mengen von 10 ppm bis 5 Gew.-% bezogen auf 6-Aminocapronsäurenitril erhalten werden.

Als Katalysatoren können bei dieser Hydrierung vorteilhaft solche auf der Basis eines Metalles ausgewählt aus der Gruppe bestehend aus, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannte Umsetzung sind beispielsweise in WO-A-96/20166, der Deutschen Anmeldung 19 636 768.9 und der Deutschen Anmeldung 19 646 436.6 beschrieben.

Adipodinitril, Aminocapronitril und Hexamethylendiamin, von denen sich Tetrahydroazepin nicht einfach abtrennen läßt, sind bedeutende Faservorprodukte zur Herstellung von Polyamiden wie Nylon-6 oder Nylon-66, die Tetrahydroazepin üblicherweise als unerwünschte farbgebende Verbindung enthalten.

Als Nitrile III kommt 6-Aminocapronitril in Betracht.

Als Amine V kommen vorzugsweise acyclische Amine, insbesondere Hexamethylendiamin, sowie deren Gemische in Betracht, wobei sich das Amin V von dem Amin II vorzugsweise unterscheidet.

Die Herstellung solcher Amine kann in an sich bekannter Weise, beispielsweise nach den für die Herstellung von Tetrahydroazepin genannten Verfahren, erfolgen.

Als Katalysatoren kommen vorzugsweise solche auf der Basis von Edelmetallmetallen, insbesondere Palladium und Platin sowie deren Gemische in Betracht. Die Katalysatoren können mit Cobalt, Eisen, Nickel, Rhodium, Ruthenium oder deren Gemische dotiert sein, vorzugsweise in Mengen von 0,1 bis 15 Gew.-% bezogen auf die Gesamtmenge an katalytisch aktiven Elementen.

Die Katalysatoren können vorteilhaft in Form von Trägerkatalysatoren eingesetzt werden, wobei sich als Trägermaterial Aluminiumoxid, Siliciumoxid, Titanoxid, Zirkonoxid und Gemische davon, insbesondere Aktivkohle empfehlen. Die Katalysatorträger können mit Verbindungen der Alkalimetalle und Erdalkalimetalle oder deren Gemische dotiert sein.

Solche Katalysatoren sind an sich bekannt.

Die Reaktion wird in Gasphase oder bevorzugt in Flüssigphase kontinuierlich an einem Festbettkatalysator durchgeführt, insbesondere in Gegenwart von 0,01 bis 2 Gew.-% Wasser bezogen auf die Summe aus Nitril III und Verbindung I. Die Reaktionstemperatur beträgt zwischen 20 und 150°C, bevorzugt zwischen 40 und 120°C. Bei der Reaktion in Gasphase wird der Druck so gewählt, daß das zu reinigende Amin/Nitril-Gemisch zusammen mit Wasserstoff in der Gasphase vorliegt. In der Flüssigphase soll der Druck zwischen 1 und 200 bar, bevorzugt 5 bis 80 bar betragen. Wasserstoff wird bei entsprechendem Druck pur oder mit mit 5 bis 95 % Stickstoff verdünnt zugegeben.

Das Amin II kann von dem Reaktionsgemisch nach an sich bekannten Verfahren, beispielsweise durch Destillation oder Extraktion abgetrennt werden. Solche Verfahren sind beispielsweise in der Deutschen Anmeldung 19 636 765.4 beschrieben.

Man erhält nach dem erfindungsgemäßen Verfahren in Gegenwart oder Abwesenheit von Hexamethylendiamin als Amin V als Amin II Azepan. Azepan läßt sich von 6-Aminocapronsäurenitril und gegebenenfalls Hexamethylendiamin gut abtrennen, so daß nach dem erfindungsgemäßen Verfahren Faservorprodukte mit hoher Reinheit und daraus Fasern mit guter Farbbeständigkeit erhalten werden können.

## Patentansprüche

1. Verfahren zur Hydrierung einer Verbindung aus der Gruppe der Imine oder Enamine I zu einem Amin II in Gegenwart eines Nitrils III, wobei das Nitril III im wesentlichen nicht hydriert wird, dadurch gekennzeichnet, daß man eine Mischung enthaltend Tetrahydroazepin als Verbindung I und 6-Aminocapronitril als Nitril III in Gegenwart eines Edelmetalle enthaltenden Katalysators IV mit einem molekularen Wasserstoff enthaltender, Gas umsetzt.

2. Verfahren nach Anspruch 1, wobei man die Hydrierung in Gegenwart eines von Amin II unterschiedlichen Amins V durchführt.

3. Verfahren nach Anspruch 1 oder 2, wobei man als Amin V Hexamethylendiamin einsetzt.

4. Verfahren nach den Ansprüchen 1 bis 3, wobei der Katalysator IV Palladium oder Platin als katalytisch aktive Komponente enthält.

5. Verfahren nach den Ansprüchen 1 bis 4, wobei man als Katalysator IV einen Trägerkatalysator einsetzt.

6. Verfahren nach den Ansprüchen 1 bis 5, wobei man als Wasserstoff enthaltendes Gas Wasserstoff einsetzt.

7. Verwendung von Katalysatoren IV zur Umsetzung von Imin 1 zu Aminen II nach einem Verfahren gemäß den Ansprüchen 1 bis 6.

## Claims

1. A process for hydrogenating a compound in the group of the imines or enamines I to an amine II in the presence of a nitrile III, said nitrile III being essentially not hydrogenated, which comprises reacting a mixture comprising tetrahydroazepine as compound I and 6-aminocapronitrile as nitrile III with a gas comprising molecular hydrogen in the presence of a noble metal catalyst IV.

2. A process as claimed in claim 1, wherein the hydrogenation is carried out in the presence of an amine V, which differs from said amine II.

3. A process as claimed in claim 1 or 2, wherein hexamethylenediamine is used as amine V.

4. A process as claimed in any of claims 1 to 3, wherein catalyst IV contains palladium or platinum as catalytically active component.

5. A process as claimed in any of claims 1 to 4, wherein a supported catalyst is used as catalyst IV.

6. A process as claimed in any of claims 1 to 5, wherein the hydrogen-comprising gas used is hydrogen.

7. The use of catalysts IV for conversion of imine I into amines II according to a process as claimed in any of claims 1 to 6.

## Revendications

1. Procédé d'hydrogénation d'un composé du groupe des imines ou énamines I en une amine II en présence d'un nitrile III, dans lequel le nitrile III n'est sensiblement pas hydrogéné, caractérisé en ce qu'on fait réagir un mélange contenant de la tétrahydroazépine comme composé I et du 6-aminocapronitrile comme nitrile III avec un gaz contenant de l'hydrogène moléculaire, en présence d'un catalyseur IV contenant des métaux nobles.

2. Procédé suivant la revendication 1, dans lequel on effectue l'hydrogénation en présence d'une amine V différente de l'amine II.

3. Procédé suivant l'une des revendications 1 et 2, caractérisé en ce qu'on met en oeuvre, comme amine V, de l'hexaméthylène-diamine.

4. Procédé suivant l'une des revendications 1 à 3, dans lequel le catalyseur IV contient du palladium ou du platine comme composant catalytiquement actif.

5. Procédé suivant l'une des revendications 1 à 4, dans lequel on met en oeuvre un catalyseur sur support comme catalyseur IV.

6. Procédé suivant l'une des revendications 1 à 5, dans lequel on met en oeuvre de l'hydrogène, comme gaz contenant de l'hydrogène.

7. Utilisation de catalyseurs IV pour la transformation d'imines I en amines II selon un procédé suivant l'une des revendications 1 à 6.
